# EUROPEAN PATENT APPLICATION

(11) **EP 1 679 365 A1**
(43) Date of publication of application: **12.07.2006**
(21) Application number: 03780967.0
(22) Date of filing: 19.12.2003
(51) Int. Cl.: C12M 3/00, C12M 1/36, C12N 5/06, G01N 33/48

(54) **CELL HANDLING DEVICE, HUMAN TISSUE REGENERATION COMPOSITION, AND HUMAN TISSUE REGENERATION METHOD**

(30) Priority: 20.10.2003 JP 2003359936; 20.10.2003 JP 2003359935
(71) Applicant: JMS Co., Ltd., Hiroshima-shi, Hiroshima 730-8652 (JP); Yuge, Rui, Hiroshima-shi, Hiroshima 731-5136 (US)
(72) Inventor: YUGE, Rui, Hiroshima-shi, Hiroshima 731-5136 (JP); MATSUURA, Yoji, Hiroshima-shi, Hiroshima 733-0034 (JP); SAJIKI, Toshinobu, Hiroshima-shi, Hiroshima 730-0005 (JP)
(74) Representative: Grünecker, Kinkeldey, Stockmair & Schwanhäusser Anwaltssozietät
(86) International application number: PCT/JP2003/016397
(87) International publication number: WO 2005/037984

(57) **Abstract**

The main objects of the present invention, which relates to regenerative medical treatments, are to enable (i) storage and conveyance of harvested or cultured cells without contamination occuring (ii) simple injection of the cells into a living body. To achieve these objects, cells harvested from a living body, or cells obtained by culturing harvested cells, are stored in a syringe-type storage vessel and subsequently transplanted into a living body. It is preferable that at least a part of the storage vessel inner wall in contact with the cells is formed from a cell non-adhesive material. Besides enabling cells in the vessel to take in the oxygen they require to survive, the present invention also enables cells quick and easy transplantation of cells into a living body without a cell detachment process, because cells are prevented from adhering to the inside of the vessel. Further, it is preferable that a stored tissue regeneration composition contains cell culture microcarriers floating in a fluidity medium, and that the cell culture microcarriers are composed of a bioabsorbable material and have cells adhering to their surfaces. Using this kind of tissue regeneration composition, a regenerative treatment can be carried out satisfactorily by simply and quickly transplanting cells from the syringe-type cell storage vessel into a living body without intricate scaffold-related procedures being required.

## Description

### Technical Field

The present invention relates to regenerative medical treatment, and, in particular, to a cell handling device and a composition used in regenerative medical treatment.

### Background Art

In recent years, on account of the progress in molecular cell biology and cell technology, research into regenerative medical treatment in which differentiated cells or self-renewing and multipotent cells (stem cells) from a living body are transplanted into a patient has continued to advance. Regenerative treatment is a method of treatment in which targeted tissue (including organ) is repaired and caused to recover by transplanting differentiated cells or stem cells into an area where tissue has been lost or into a part of the body responsible for causing disease. Generally, in regenerative medical treatments, extracted cells are cultured independently, or using scaffolds, for a short time in a vessel designed for the purpose. The differentiated or yet to be differentiated cells obtained via this procedure are then transplanted to the targeted region via a surgical procedure.

In this type of regenerative medical treatment, invasive surgical procedures for transplanting the cells are often performed. As the burden of such techniques on the patient is great, methods for injecting cultured cells directly into the body are being investigated. The injection of cartilage cells or their progenitors into joints, the injection of nerve cells or cells that produce physiologically active substances, or their progenitors, into the brain, and the injection of cardiac muscle cells or their progenitors into the heart are examples of treatment methods that are likely to be influential. When such treatments are implemented, cells attached to the wall of the vessel are detached using trypsin, EDTA (ethylenediaminetetraacetic acid, commonly known as edetic acid) or the like, and a prescribed quantity of cells obtained via a washing or similar process. Subsequently, the cells are commonly injected into the body using a syringe or a catheter.

Moreover, in the cell transplantation of regenerative medical treatments, not only is the series of procedures, including cell harvest, culture, differentiation inducement and transplantation into the body, comparatively intricate, but, in order to prevent contamination, each procedure must be carried out under clean conditions, and advanced techniques requiring skill and experience must be employed. Consequently, for anyone not trained in advanced techniques or in possession of a well-equipped facility, cell transplantation treatments are difficult to implement. Further, in addition to the difficulties of the procedures, there is the further difficulty of conveying the harvested or cultured cells to the prescribed clean room, incubator equipment, or the like without contamination from the surrounding environment.

### Disclosure of the Invention

The present invention was conceived in the light of the above problems and has the principal objects of enabling harvested or cultured cells to be conveyed and stored without contamination from the surrounding environment, and of enabling the cells to be easily injected into a body.

However, since during research directed towards achieving these objects the following problems were found to exist, the present invention has additional the object of solving these problems.

Namely, in conventional regenerative medical treatments, no cell handling device has been conceived to enable anyone to simply perform the series of operations in a treatment, including the harvesting, preservation, culture, and simple transplantation of cells into a living body.

Specifically, since many cells cannot survive or proliferate unless adhering to a scaffold, the cells adhere to the wall of the culture vessel, using it as a scaffold. Hence, when the cells are to be transplanted into a living body, the cells adhering to surfaces in the vessel must be detached. To do this, operations including physical detachment and detachment using a chemical agent such as trypsin and/or EDTA can be used, but these operations can adversely affect the cells. Furthermore, these types of cell operations are difficult to implement for anyone not in trained advanced techniques and not in possession of a well-equipped facility.

Hence, the present state of affairs, in which cell transplantation cannot be carried out easily, can be said to be a result of having to detach the cultured cells from the walls of the vessel.

Consequently, another object of the present invention is to provide a cell handling device that, while having a simpler construction than conventional equipment, (a) enables cells to be stored satisfactorily while preventing contamination, and (b) at cell transplantation, enables cells to be injected into a living body in a simpler way without a process to detach the cells from the vessel.

The principal object of the invention is achieved via a tissue regenerative method in which cells harvested from a living body or cells obtained by culturing such cells are stored using a syringe-type device (i.e. applying a force to a piston manually, via control of air pressure or other mechanical means to change the volume of the liquid storage space therein causes inflow and outflow (applying a pressure causes the device to discharge its contents from the discharge opening)) as the cell handling device, and the cells stored in the device are transplanted into a living body.

Thus, by using the syringe-type cell handling device, at cell transplantation in a regenerative treatment, cells can be transplanted to a living body via an operation similar to that employed for normal medical-treatment-use syringes. Hence, since cell transplantation can be carried out comparatively simply and quickly and is not limited to highly trained operators with special skills, this method is advantageous. The syringe-type cell handling device has a further advantage in that the influx and efflux of the contained substance can be easily controlled by controlling the rate of change of internal pressure.

In order to achieve the aforementioned objects, inventions relating to the types cell handling devices and tissue regeneration compositions described below were produced. A combination of these is extremely effective in terms of achieving the principal objects, which are to enable the culture, storage and conveyance of cells without contamination from the surroundings, and to enable cells to be easily injected into a living body.

In order to achieve the principal objects the cell handling device of the present invention was given a construction which includes a vessel able to hold, in a liquid-tight state, a handling medium that is fluid and contains cells, and is able to transfer the handling medium between an interior and an exterior of the vessel via a mouth being opened in the vessel to end the liquid-tight state, the mouth connecting the interior and the exterior, wherein at least part of the vessel that contacts the handling medium when the vessel holds the handling medium is a gas permeable region for passing a quantity of gas necessary for survival of the cells.

Here the "region allowing gas to permeate (gas permeable region)" indicates a region that is permeable by a gas in areas coming into contact with the cells and that is formed from a material which is impermeable by liquids. Note, however, that when a gas permeable region is provided in areas that do not come into contact with cells, it is not required to be liquid-tight.

The gas permeable region of the present invention is a region with an oxygen permeability of at least 0.1 mL / cm² 24 hr atm. There is no particular limit on the area of the gas permeable region. However, from the point of viewof supplying the cells with suf f icient quantities of the oxygen they require, in sections where the device is in contact with the cell suspension, an overall oxygen permeability of at least 1 mL / 24 hr atm is favorable, and 10 mL / 24 hr atm especially favorable.

Hence, in the cell handling device of the present invention, since gas circulation (gas exchange) is possible via the gas permeable region, even when material impermeable by both gases and liquids is used for other parts of the device, by taking in oxygen necessary for cell survival, by exhausting carbon dioxide, and the like to regulate the gas concentrations, maintenance of conditions such as the pH of the culture fluid in the suspension is realized while the cell suspension is kept sealed therein. Hence, while preventing the cells harvested for regenerative medical treatment from deactivating inside the device, it is possible both to convey the device and to have the cells proliferate satisfactorily or induce them to differentiate.

If the gas permeable region is provided throughout the cell storage part of the cell handling device, a uniform and sufficient gas exchange becomes easier to achieve with respect to the entire body of cells in the suspension. Hence, even when the gas permeable material is not particularly gas permeable, a sufficient level of gas exchange can be achieved.

If, on the other hand, a material with a gas permeability that is comparatively favorable is used, a construction in which this material is provided throughout the cell handling device is unnecessary, and it is acceptable, instead, to provide the gas permeable material in the reservoir section of the handling device across at least a part of the inner wall that is in contact with the suspension. In this case, the gas permeable region may, for example, be rectangular, circular, or another shape, and have a predetermined area.

In the case of a cell handling device of the syringe-type, it is desirable to form a gas permeable region across all or a portion of the main body part storing the cells and across a portion of the plunger. Since when a gas permeable region is formed across a portion of the main body it will have a limited area, designing a device
in which a material with a comparatively high permeability is used so that sufficient gas for the survival of the cells can be secured is considered to be desirable. Note that the degree of permeability will depend on the minimum gas concentration (oxygen, carbon dioxide) needed for the survival of the cells. In other words, though the amount of gas needed for the survival of the cells is different according the type of cells, in order to have the cells survive, it is preferable that a material with a high permeability is used and that a sufficient level of gas exchange takes place.

Further, for reasons such as the limited choice of materials, when the gas permeable region is to be formed across a portion of the body, providing a plurality of separate independent permeable regions is desirable because this results in an improvement in both the uniformity and sufficiency of the supply of gas across the whole cell reservoir section.

One material with superior gas permeability, which is used when the gas permeable region is provided across a portion of the main body, is porous film. By controlling the diameter of the pores of this porous film, impermeability with respect to liquids can be maintained. On account of this it has been discovered that if a film whose pores are formed to sufficiently small to guarantee its impermeability with respect to liquids is used, sufficient gas permeability can be ensured, even if only a comparatively small area of the material is provided.

Further, if a porous film is used as the gas permeable region, bubbles that exist in the cell suspension stored in the cell handling device can be safely exhausted out of the device through the porous film.

In the case of syringe-type cell handling devices, examples of possible forms for the main body include (i) a form in which cells are stored directly in a cylindrical vessel (an outer cylindrical body), and (ii) a form in which a bag-type vessel, such as a concertina form vessel, a bag form vessel, or a tube form vessel, whose internal space can be reduced via the application of a pushing force, is housed in an external body. In the former(i), forming the gas permeable regions in, say, the main body, the cap for sealing the vessel with respect to liquids, or in the plunger is desirable. In the latter (ii), forming a gas permeable region in at least one portion of the internally housed bag-type vessel to enable gas to be supplied to the cells is desirable. Further, in particular, in the latter(ii), when the bag-type vessel is housed in the outer cylindrical casing, forming gas permeable regions in areas besides the bag-type vessel (for instance, the plunger, the external cylindrical casing, and the like) to enable gas exchange between inside the bag-type vessel and the device exterior is desirable. Further, while the bag-type vessel can naturally store cells and have them proliferate in its attached state, being detachable, it is further capable of storing cells and having them proliferate in its detached state. This is because, as well as being liquid-tight, the bag-type vessel itself provides gas permeability.

Further a film composed of a macromolecular material with favorable gas permeability may be used as the film forming the gas permeable region. Even when a macromolecular material whose gas permeability is not particularly favorable is used in this capacity, if made into a porous film and provided with appropriately sized holes, it can be made gas permeable but impermeable by liquids. Thus, it is possible to make the cell handling device liquid-tight and prevent leakage of any part of the cell suspension from the gas permeable portion.

Further, in the present invention, forming sections of the cell handling device in contact with the cell suspension from a material that cells have difficulty adhering to is effective. There are various methods for evaluating the adhesiveness of the cells, including the detection of assisting proteins that form focal contacts (desmosomes) using methods from immunology and counting of the number of adhering cells.

Decreasing in the adhesiveness of the internal surfaces of the cell handling device in this way enables the adhesion of cells to the internal walls of the cell handling device to be suppressed during the period that the cells are stored. This enables the following practical benefits to be obtained during a regenerative treatment.

Conventionally, cells to be transplanted in a regenerative medical treatment were cultured and stored using, for example, a culture-use petri dish, or the like. However, since, in this type of conventional cell handling device, cells adhered to the internal surfaces, some processing to detach the cells was necessary when transplantation to a living body took place. Further, in order to carry out this detachment processing, a highly skilled and experienced operator working with equipment that strictly prevents contamination was required, and regenerative medical treatment could not be carried out easily. With the cell handling device of the present invention, on the other hand, detachment processing is not required when the cells are removed from the cell handling device, and since cells can be transplanted undamaged to a living body without using either physical detachment methods or drugs, satisfactory implementation of regenerative medical treatments can be anticipated. Further, as carrying out the detachment process is unnecessary and the cells stored in the cell handling device can be transplanted as they are into a living body, the complexity of the transplantation can be reduced and even an operator who has special expertise can easily transplant cells.

Moreover, the present inventors pursued research based on their own ideas about the form and material of the scaffold used to have cells proliferate and induce dif ferentiation in cells for regenerative medical treatments, and by the combined actions of setting the shape of the scaffold to a be grain-like shape and forming the scaffold from a material that is bioabsorbable, they were able to demonstrate a great simplification in the operations associated with cell culture, and this led them to develop the tissue regeneration composition of the present invention. Specifically, the tissue regeneration composition of the present invention includes a fluidity medium and cell scaffoldmicrocarriers, granular in form, which become scaffolds for the cells, the cell scaffold microcarriers being composed of a material that is bioabsorbable, and the cells adhering to the cell scaffold microcarriers.

Using this type of tissue regeneration composition, cells harvested from a living body can be made to proliferate, or induced to differentiate to become target cells, on the surfaces of the scaf fold microcarriers. During this period, the cells adhere to the scaffold microcarriers and have fluidity because of the fluidity medium (a culture fluid (including a humor)). At transplantation, the cell-matrix complex is injected as they are into a living body.

Hence, none of the conventionally required cell detachment processing is necessary after cell proliferation, and a great improvement is possible in the efficiency of the cell transplant operations in regenerative medical treatments. Further, since no cell detachment processing takes place, there is no need to worry about damaging the cells. Note here that the tissue regeneration composition injected into the affected part does not necessarily have to contain cells that adhere to the scaffold microcarriers. It is also possible to inject only the scaffold microcarriers, and use them as scaffolds for the cells of the host.

Further, as the scaffold microcarriers are formed from a bioabsorbable material, they are absorbed into the living body and disappear a predetermined period after being injected. Consequently, there is no need for a second operation to remove the scaffold.

For the transplantation of cells into a human body, a method was attempted in which cells suspended in a culture fluid were transplanted, but with this method, as a result of the high fluidity of the suspension, the cells were carried away, and had difficulty attaching and surviving in the transplantation target area. In order to solve this problem, trials were carried out using a method in which cells for transplantation were dispersed in a dispersion matrix (a high viscosity solution such as gelatine or collagen, for example) so as not to be carried away, and the obtained suspension (high viscosity solution) was transplanted to the affected area. However, in this method, the dispersion matrix acted as a barrier and the cells did not attach and survive very well in the transplantation target area. In the tissue regeneration composition described above, on the other hand, the cells adhere to the surfaces of the microcarriers. Thus, when used in cell transplant trials, problems of the type described above do not readily occur, and the cells are found to attach and survive satisfactorily.

Moreover, since the scaffolds are grain shaped, surface area per unit volume is high, and many cells can therefore be made to adhere to a small quantity of scaffolds.

Further, if a tissue regeneration composition made up of these type of cell culture microcarriers is stored in the syringe-type cell handling device described above, at transplantation, cells can be simply and quickly transplanted from the cell handling device into a living body. Consequently, this combination enables both a reduction in the amount of surgery on patients who lack physical strength such as children and the elderly, and a reduction in the burden on patients of regenerative treatment in general.

Moreover, storing the tissue regeneration composition in the above described cell handling device in this way has the beneficial effect of enabling the stages of the process - cell conveyance, culture and transplantation into a human body - to be linked, and implemented simply using a single vessel. More specifically, one beneficial effect is that the operation of inserting the cells into a specialized culture vessel can be omitted because a gas permeable region is provided in the above-described cell handling device, enabling cells to be both stored and cultured therein. An additional beneficial effect results from the syringe format of the device which enables cells stored and cultured therein to be transplanted as they are, using the device, via a duct such as a needle or catheter.

When a transplant is implemented using the above-described device, if the cells adhere to the walls of the device, the number of injected cells is reduced, and cells may not be transplanted into the body in sufficient quantities. In order to solve this problem it is effective to give the device the above-described characteristic of internal walls that are non-adhesive with respect to cells, and reduce the number of adhering cells. This, however, results in the loss of scaffolds required by the adhesive cells during culture/differentiation. In such a situation, using the bioabsorbable scaffold microcarriers is very effective, because they offer scaffolds for this type of adhering cell.

Note here that, because when there are a plurality of sections for the cells to adhere to, a cell will tend to adhere to the section which is easiest to adhere to, it is obvious that, in order for the scaffold microcarriers to fulfill their function, the scaffold microcarrier material must be easier for the cells to adhere to than the internal walls of the device.

### Brief Description of the Drawings

FIGs. 1A-1C are structural drawings of a syringe that is the cell handling device of the First Embodiment;
FIG. 2 is a structural drawing of a syringe that is the cell handling device of the Second Embodiment;
FIG. 3 is a structural drawing of a syringe that is the cell handling device of the Third Embodiment;
FIG. 4 is a structural drawing of a syringe that is the cell handling device of the Fourth Embodiment;
FIG. 5 shows performance data for the cell handling devices of the present invention;
FIG. 6 is a structural drawing of the cell handling device of the Fifth Embodiment;
FIG. 7 is a structural drawing of the cell handling device of the Sixth Embodiment;
FIG. 8 is a structural drawing of a syringe that is the cell handling device of the Seventh Embodiment;
FIG. 9 is a structural drawing of a syringe that is the cell handling device of the Eighth Embodiment; and
FIG. 10 is a structural drawing of the tissue regeneration composition of the present invention.

### Best Mode for Carrying Out the Invention

Firstly, the tissue regeneration method of the present invention is explained.

The tissue regeneration method of the present invention essentially includes the following steps.
i. Harvesting step of harvesting cells from a living body
   First, specified cells are harvested from a living body.
ii. Isolation and purification step
   Harvested cells are isolated and purified using FACS (flow cytometry) or the like.
iii. Storing step of storing cells in the cell handling device
   Next, the cells are stored in the cell handling device
iv. Proliferation step
   The cell handling device storing cells is stored in a cell processing center (CPC). Next, in the cell processing center or the like, the cells inside the cell handling device proliferate, and where necessary, are induced to differentiate.
v. Transplantation step
   Using the cell handling device, the cells that have differentiated and proliferated are transplanted into a living body.

Here, in steps iii. tov. , by using the syringe-type cell handling device indicated in the First to Eighth Embodiments below, the storage, conveyance, cell proliferation and transplantation processes of a regenerative medical treatment can be treated as a single linked process using the same device. Consequently, there is no need to transfer cells to another vessel, and it is possible to proceed to subsequent processes quickly and safely, and to treat patients quickly.

The cell handling device is particularly useful at cell transplantation on the scene of a regenerative treatment. Stored cells can be transplanted into a living body with an operation similar that of a conventional medical-use syringe. Consequently, cell transplantation can be carried out simply and is not limited to highly trained operators. Further the cells can be conveyed while they are stored.

Further, on the scene of a regenerative treatment, the locations at which the various steps take place are often situated apart from one another, but since the cell handling device of the current invention can be handled while having liquid sealed therein, it is suitable for conveying the cells between the locations.

For example, the cells can be handled simply and quickly by using the device to store cells in the storing step of iii. for the period between the cells being harvested from a living body and the cells being cultured or being transplanted, as described above. Alternatively, the cell handling device of the present invention can be used when cells are cultured in the proliferation step of iv. Further, the device can be used as a cell culture device and then used in its existing state as a means to store the cells.

A cell suspension generally includes a culture liquid and cells. A conventional cell culture liquid can be used in its existing state, but when the culture liquid is to be transplanted together with the cells into a living body, safety considerations make it desirable to reduce as far as possible, or eliminate, the addition of materials (viruses, prions) that can cause infection.

Here, the cell suspension can be made to include the various type of cells used in regenerative medical treatments. The cells can be any of the various types described above depending on the aim of the treatment. There are no particular limits to the type of cell that can be used and besides stem cells, differentiated cells or their progenitors can be used. Some examples of stem cells that can be used are embryrionic stem cells (ES cells), embryonic germ cells (EG cells), adult stem cells (AS cells), mesenchymal stem cells, neural stem cells, endothelial stem cells, hematopoietic stem cells, and hepatic stem cells. Examples of the differentiated cells include bone cells, chondrocytes, muscle cells, heart muscle cells, nerve cells, tendon cells, fat cells, pancreatic cells, heptocytes, liver cells, hair follicle cells, blood cells and the like. Thus, embryonic stem cells and other stem cells at various stages of differentiation, and cells that have differentiated to form various tissues can be used. Of these, when adhering cell types are used, making the cell handling device non-adhesive with respect to cells and using fine grained scaffolds are effective. This is because adhering cells require scaffolds for proliferation and differentiation.

These types of cell can be harvested using a well-known method, in which tissue (including cells) is separated from a predetermined area of a living body, the required cells are selectively separated from the separated tissue, growth factor, cytokine, or the like is then added as required, and the cells are cultured. It is desirable to implement the culture inside a dedicated incubator. In this method, the cultured cells are stored inside the syringe-type cell handling device under appropriate conditions until they are required for a treatment.

The following are examples of cells and culture liquid combinations.

When human mesenchymal stem cells are used in the cell culture, the culture liquid can be produced by adding mesenchymal stem cell growth supplement (50 mL), L-Glutamine (10mL) and penicillin/streptomycin (0.5 mL) to 440 mL of human mesenchymal stem cell basal medium (POIETICS Ltd., USA).

Further, when chondrocytes are used, dexamethasone (1 mL), sodium pyruvate (2 mL), ascorbate (2 mL), proline (2 mL), ITS + supplement (2 mL), L-Glutamine (4 mL) and penicillin/streptomycin (2 mL) added to hMSC differentiation basal medium (185 mL) can be used as the cartilage differentiation inducing culture site (culture liquid).

The tissue regeneration composition that contains the cell culture microcarriers of the present invention, meanwhile, is described in detail below.

The following detailed description is for when a syringe-type cell handling device is used and a cell suspension stored therein.

### 1. Cell handling device of the present invention

### (First Embodiment)

### 1-1. Construction of syringe-type cell handling device 1

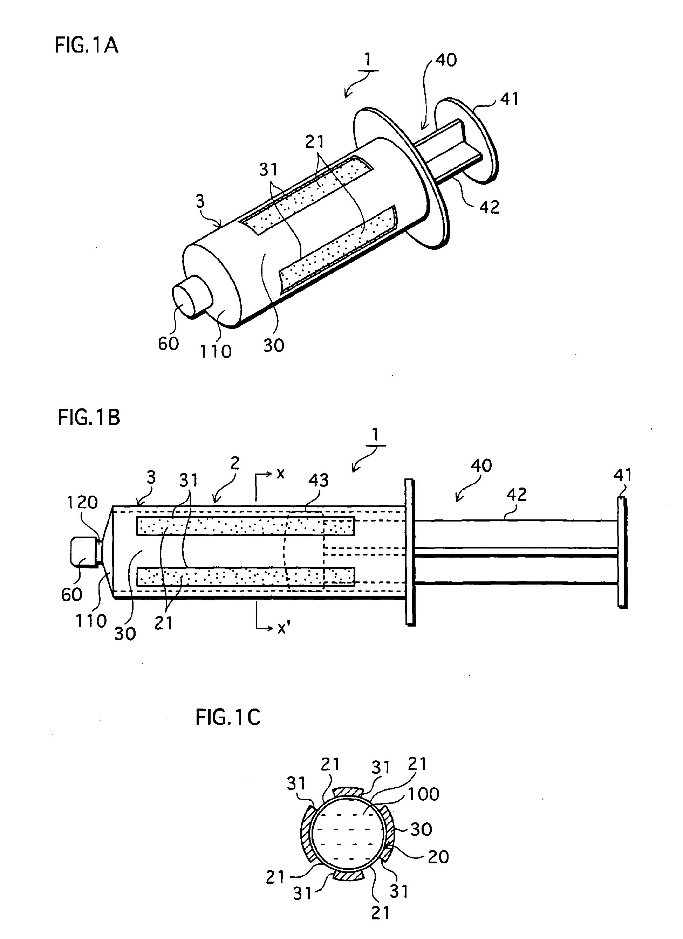

FIGs . 1A-1C show the structure of syringe-type cell handling device 1 of the First Embodiment that is one example of the cell handling device of the present invention. FIG. 1A is a perspective view, FIG. 1B is a side elevation, and FIG. 1C is a cross-section through X-X' of FIG. 1B.

The syringe-type cell handling device 1 shown in FIG. 1 is broadly composed of a syringe main body 2 and a plunger (also referred to as a pressing component or as a piston) 40. A cell suspension 100 is held within the syringe body 2.

The syringe main body 2 is composed of cylindrical body 3 made by injection molding a material that is non-adhesive with respect to cells to form a cylinder, and a gas permeable film 20 which is described below.

The cylindrical body 3 is formed with a leur 120 protruding from a disk shaped front section 110 at a front-end surface. Under normal conditions, a cap 60 is fitted to the tip of the leur 120. The plunger 40 is inserted from the back-end 12 side of the syringe main body 2, thereby making the internal part of the syringe main body 2 liquid-tight. Note that the leur 120 may alternatively be sealed using a resin or the like, the seal to be broken when the device is used (at cell transplantation).

As long as it can be shaped, any material can be used for the cylindrical body 3, including any of the materials commonly employed to make syringes. However, from the point of view of realizing one of the characteristics of this invention, using a material that is difficult for cells to adhere to is preferable. As described in detail below, by using this cell non-adhesive material for a portion of the cylindrical body 3, cells can be prevented from adhering to the insides of the cylindrical body 3, and during culture, be stored a favorable manner, floating in the culture liquid.

Here, "cell non-adhesive" means either that cells do not adhere to the walls at all, or that they adhere to some degree but are easily detached.

The plunger 40 is injection molded from a material such as polyethylene, polypropylene, polycarbonate, polyvinyl chloride or the like, and has a construction in which disk shaped end parts extend in a radial direction from both ends of a main body 42 having a cross-shaped profile. One of these end parts is a plunger head 43, which is inserted axially into the syringe main body 2. The other end part is a pushing end 41, which the user pushes with his fingers in order to push the plunger 40 into the syringe main body 2.

The plunger head 43 is constructed from an elastic material and arranged so that the cell suspension 100 can be held liquid-tight within the syringe main body 2 (specifically, the cylindrical body 3 and the gas permeable film 20).

### (Cell non-adhesive material)

Here, "an evaluation of whether or not it is difficult for the cells to adhere to", which may be rephrased as "an evaluation of whether cell non-adhesiveness is present", can be carried out by detecting supporting proteins forming focal contacts (desmosomes) via methods from immunology, counting the number of adhering cells, or the like.

Some preferred options for the cell non-adhesive material are described below. Although the best material will vary according to the type of cell to be stored, preferable materials include certain hydrophilic materials, certain hydrophobic materials, and certain materials with a negatively charged surface, all of which are cell non-adhesive. A material with an angle of contact with respect to water of not more than 50 degrees is preferable as the hydrophilic material, and a material with an angle of contact of not less than 100 degrees with respect to water is preferable as the hydrophobic material.

Examples of preferable hydrophilic materials include any of a number of materials that are coated, or bonded using methods such as graft copolymerization or chemical reaction, onto the surface of a base material. Examples of preferable hydrophilic materials include acrylamide copolymer, methacrylamide copolymer, polyacrylic acid, polyvinyl alcohol, polyethylene glycol, polyvinyl pyrrolidone, cellulose, dextran, hyaluronic acid, glycosaminoglycan, proteoglycan, carrageenan, and proteins. The adjustment and coating processes for the surface of the base material must be carried out separately, after the manufacture of the base material by injection molding or the like.

Examples of possible hydrophobic materials include fluoropolymers suchas polytetrafluoroethylene, tetrafluoroethylene -hexafluoropropylene copolymer, polyethylene terephthalate, polypropylene and the like, and silicone resins.

Further, examples of material having a negative charge at the surface include materials with polyacrylic acid, polymethacrylic acid, styrenesulphonic acid, alignic acid, heparin, heparan sulfate, chondroitin sulfate or dermatan sulfate bonded to the surface thereof. Of these, materials containing carboxyl groups are preferable because the smoothness of such materials gives superior non-adhesiveness with respect to cells.

Of all the materials described above, silicone resin is preferable because it also has excellent gas permeability. Further, the copolymers polytetrafluoroethylene and tetrafluoroethylene-hexafluoropropylene are similarly preferable because a high gas permeability can be obtained by making them into porous films.

Note also that is desirable to form a gasket that is in contact with the cells using the material that is cell non-adhesive.

### (Gas permeable region)

In surrounding walls 30 of the cylindrical body 3, through holes are provided in a thickness direction of the cylindrical body 3. Here, as shown in FIG. 1A, through holes (here, four) are provided as rectangular shaped slits 31 with a length direction in the syringe axial direction. However, the form of the rectangular shaped slits 31 is only one example of a possible form for the through holes, and the form and number of through holes are not limited by this example.

Further, in the slits 31, as shown in FIG. 1C, a cylindrical gas permeable film 20 is provided in contact with the internal surfaces of the syringe main body 2, the film extending along the entire length of the body 2 except for the leur 120. The gas permeable film 20 can be constructed from a material with a higher gas permeability (for example, a film composed of a gas permeable material) than the principal material of the slits 31. With the gas permeable film 20 covering the rectangular slits 31, portions of the film exposed to the exterior through the rectangular slits 31 form a gas permeable region 21 composed of a plurality of gas permeable region units.

Note that the gas permeable film 20 need not be cylindrical, but can also be provided by combining strips of film and the exterior side of the cylindrical body 3 so as to cover each of the slits 31, making them liquid-tight.

As the above gas permeable material, a gas permeable resin that is impermeable by the suspension 100(one able to hold the suspension 100 liquid-tight inside the device) can be used. This gas permeable resin may be, for example, silicone resin, poly-4-methyl-1-pentene (P4M1P), polyisoprene, polybutadiene, ethylene vinylacetate copolymer, low density polyethylene, polystyrene, or the like. Though, for plastic materials, these gas permeable materials have comparatively favorable gas permeability, it falls as their thickness increases. On account of this, a thickness for the gas permeable film 20 of not more than 200 µm is generally desirable, and not more than 100 µm preferable.

Alternatively, the gas permeable material can be a porous film provided with holes of not more than a prescribed diameter, so that both leakage of the cell suspension 100 to the exterior and contamination of the cell suspension due to the intrusion of bacteria can be prevented. In this case, a hydrophobic macromolecular material is desirable as the source material for the porous film. Setting a hole diameter of not more than 1 µm is desirable, and one of not more than 0.4 µm preferable. As the hydrophobic material, polytetrafluoroethylene (PTFE), tetrafluoroethylene-hexafluoropropylene copolymer, polyethylene terephthalate (PET), polypropylene (PP), polyethylene (PE), or the like can be used. A hydrophobic polyvinylidene fluoride can also be used.

Here, as the material for the gas permeable film 20, using a hydrophobic material that is a porous film or a gas permeable resin material such as a silicone resin, polyethylene or polystyrene is preferable because the gas permeable film 20 can be made to be both gas permeable and cell non-adhesive. Of the hydrophobic materials described above, many are found to be cell non-adhesive.

Further, it is preferable that the syringe-type cell handling device 1 undergoes a sterilization process before storing the cell suspension 100. During cell storage, the cell suspension 100 is held inside the gas permeable film 20, which is inside the cylindrical body 3 of the syringe main body 2, between the plunger head 43 and the leur 120 (see FIG.1C). The cell suspension 100 may, for instance, be introduced into the syringe main body 2 via the leur 120, and then held liquid-tight therein by sealing the leur 120 with the cap 60 or a resin. Alternatively, the tip of the leur 60 can be pre-sealed, and the cell suspension 100 introduced from the back end 12 side of the cylindrical body 3.

Note that, though not shown in the drawings, the syringe-type cell handling device 1 may be allowed to hold small quantities of gas together with the suspension 100. However, excluding such gas as far as possible is desirable because, if it should form bubbles and become mixed into the culture liquid, it can damage cells.

### 1-2. Effects of the syringe-type handling device 1

The syringe-type cell handling device 1 of the First Embodiment has the cylindrical body 3 composed of a material that is impermeable by the cell suspension 100. The slits 31 are formed in the surrounding walls 30 of the cylindrical body 3, and the gas permeable region 21, composed of a material that is impermeable by the cell suspension 100 but permeable by gases, is formed in these slit areas. Hence, as the cylindrical body 3 need not be gas permeable, it can be manufactured simply using a normal injection molding process, or the like, from any of the various types of materials described above. Further, ensuring that the device has the strength and rigidity necessary in a syringe is easy.

Further, the syringe-type cell handling device 1 holds the cell suspension 100 inside the syringe main body 2, as described above, in such a way that the cell suspension 100 is able to exchange gases with the exterior through the gas permeable region 21. Consequently, as the oxygen necessary for the survival of the cells can be taken in from the exterior of the syringe-type cell handling device 1, and the carbon dioxide dependent culture liquid pH can be kept constant, the cells can be satisfactorily stored, and a reduction in cell activation inhibited.

When a cell handling device is being carried, for example, bubbles existing in the cell suspension 100, come into contact with the cells, and this can cause the destruction of the cells. In the syringe-type cell handling device 1 of the First Embodiment, however, when a porous film is used in the gas permeable areas, cells can be stored safely rather than being destroyed, because the gas permeable region 21 enables the bubbles to be exhausted to the exterior and disposed of while preventing contamination.

Moreover, though the syringe-type cell handling device 1 is capable of gas exchange with the exterior due to the provision of the gas permeable region 21, it is formed so as bacteria do not invade through the gas permeable region 21 into the syringe main body 2. Thus contamination is prevented and the cells can be stored satisfactorily. The degree to which contamination is prevented can, as discussed above, be adjusted as appropriate. When a gas permeable material is used as the gas permeable film 20, for example, adjustment is achieved by adjusting the thickness of the material and, when porous film used, by appropriately setting the diameter of the holes, or the like. Note, however, that the degree of permeability required by the cell handling device must be taken into consideration when such adjustment is carried out.

The effects of the gas permeability provided by the cell handling device of the present invention are especially needed in regenerative medical treatments when, for example, cells are harvested outside a hospital and have to be safely conveyed to a specialist facility where there is clinical testing equipment, and the syringe-type cell handling device 1 of the First Embodiment shows itself to be effective as a cell handling device to satisfy this need.

Moreover, because the syringe-type cell handling device 1 is made in the form of a syringe for medical use, when used in a regenerative treatment, the leur 120 can be connected, after removal of the cap 60, to a needle, an intravenous catheter, or other conduit. Further, the cells can be injected into a living body simply by applying finger pressure to the pushing end 41 of the plunger 40. Hence, via an operation resembling a normal syringe operation, even an operator not specially trained and not in possession of advanced techniques can, without an intricate operation being required, inject cells into the treatment area, and the regenerative treatment can thereby be implemented easily and quickly.

Further, an additional benefit is that, when the gas permeable region is formed from a porous film, bubbles can easily be expelled from the suspension 100 through the gas permeable region by pressing the pushing end 41 of the plunger 40 towards the leur 120 side of the device, and applying a pressure to the cell suspension 100.

Further, in the syringe-type cell handling device 1, when one or both of the gas permeable region 20 and the cylindrical body 3 of the syringe main body 2 are constructed from a cell non-adhesive material, the cells do not adhere to a part, or all, of the inner walls in contact with the cell suspension 100. Thus, as a result of being stored floating in the culture liquid, no process is required to detach the cells from the inner walls of the cylinder body 3 when they are transplanted to a living body from the syringe-type cell handling device 1, and the corresponding complexity of the detachment process can be eliminated. Also, since the cells are floating in the culture liquid the cells together with the culture liquid can be transplanted smoothly from inside the syringe-type cell handling device 1 into a living body by simply pushing in the plunger 40.

Moreover, because the syringe-type cell handling device 1 uses the cell non-adhesive material in the syringe main body 2, beneficial effects, such as being able to avoid, at a fundamental level, various problems associated with the conventional cell detachment, are achieved. These problems include: cell damage in the case of physical detachment of cells; harmful effects on the living body receiving the transplant due to the detachment agent in the case that pharmaceuticals (detachment agents such as trypsin, EDTA and the like) are used in the detachment process; and other intricate processing problems associated with cell detachment processes that depend on temperature modification, the need for cell cleaning processes, and the like.

If the syringe-type cell handling device 1 of the First Embodiment is used, the operation of transplanting cells on the scene of a regenerative treatment can be carried out very simply, quickly and with a high degree of accuracy. Consequently, regenerative treatments can be satisfactorily implemented while the occurrence of problems such as contamination is avoided, even in a facility with equipment that is not particularly advanced. As such, the cell handling device of the present invention satisfactorily meets the necessary conditions relating to operations in this type of regenerative treatment.

As described above, the syringe-type cell handling device 1 is applied to inject cells stored therein into the part of the living body to be treated, or into blood vessels, in treatments for osteoarthritis, rheumatoid arthritis, pseudoarthrosis, progressive muscular dystrophy, myocardial infarction, strokes, Parkinson's disease, spinal cord damage, tendon damage, diabetes, liver damage, digestive organ dysfunction, skin damage, leukemia, vascular disease and the like.

Note that though cells are conventionally cultured and stored together with culture liquid (or culture medium), when the cell suspension is injected, in its existing state, into a living body using the syringe-type cell handling device 1 of the First Embodiment, a culture liquid composition that is safe for the living body must be chosen.

For example, when injecting chondrocytes or their progenitor cells into an osteoarthritis patient, neurons or their progenitor cells into a patient with Parkinson's disease, or cardiac muscle cells into a patient with a coronary disease, and in general, when injecting cells into a human being, it is preferable to use a culture medium of the patients own serum, or a culture medium that does not contain constituents, such as bovine serum, which derive from other animals.

### (Second Embodiment)

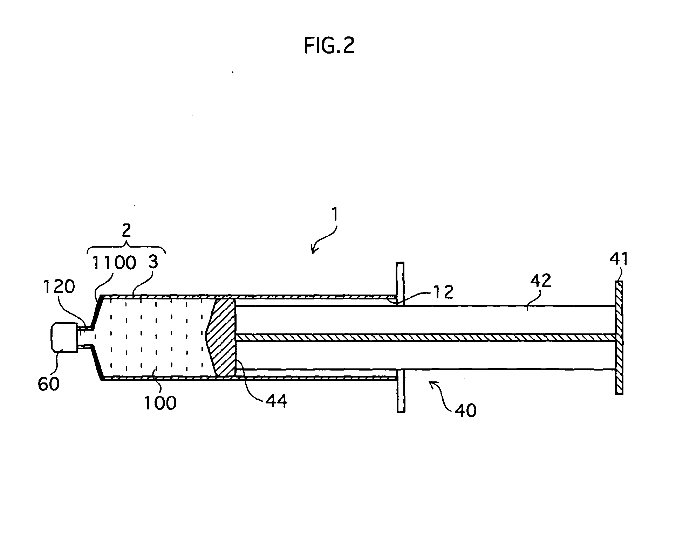

FIG. 2 is a cross-sectional drawing showing the construction of a syringe-type cell handling device 1 of the Second Embodiment that is an example of a cell handling device of the present invention. The differences from the First Embodiment are that through holes are not provided in the surrounding walls of a cylindrical body 3 in a syringe main body 2, and that a gas permeable region is formed instead in a front section 1100 of the cylindrical body 3. Specifically, this gas permeable region may be formed when forming the syringe main body 2 by using a gas permeable material to make the front section 1100, or by opening through holes in the front section 1100 and fusing or sticking a gas permeable film over the through holes, thereby making the front section 1100 liquid-tight.

The same type of cell non-adhesive and gas permeable materials as in the First Embodiment can be used. Generally, the tip of a leur 120 has a cap 60 fitted, as shown in FIG. 2, or contains a resin seal to keep the internal part of the syringe-type cell handling device 1 liquid-tight.

With the syringe-type cell handling device 1 of the Second Embodiment having the above described construction, effects (the gas exchange characteristic, the elimination of bubbles from the cell suspension 100, the capability to transplant cells simply and quickly, and the like) similar to those of the First Embodiment are achieved.

In addition, in the syringe-type cell handling device 1 of the Second Embodiment, though a large portion of the inner walls of the syringe main body 2 are in contact with the cell suspension, the cells do not adhere to the walls and can be effectively held floating the culture liquid because the cylindrical body 3 is constructed from a cell non-adhesive material.

Moreover, in the syringe-type cell handling device 1 of the Second Embodiment, unlike in the First Embodiment, through holes are not provided in the inner walls of the cylinder shaped syringe main body 2. Consequently, when eliminating bubbles, having the leur 120 pointed upwards and the plunger 40 pushed towards the leur 120 end of the internal part of the syringe main body 2 is preferable, since, if this is the case, the bubbles can be easily collected in proximity to the front section 1100, and removed therefrom while leakage of liquid is kept to a minimum. Thus, another beneficial effect of this device is to enable cells to be satisfactorily transplanted into a living body while preventing bubbles from becoming mixed into the cell suspension 100.

Note that the plunger head 44 may also be constructed from a gas permeable material. Such a construction enables gas exchange between the cell suspension 100 and the exterior to take place more satisfactorily, and is therefore desirable.

Further, constructing the plunger head 44 from a cell non-adhesive material to prevent the cells from adhering thereto is also favorable.

### (Third Embodiment)

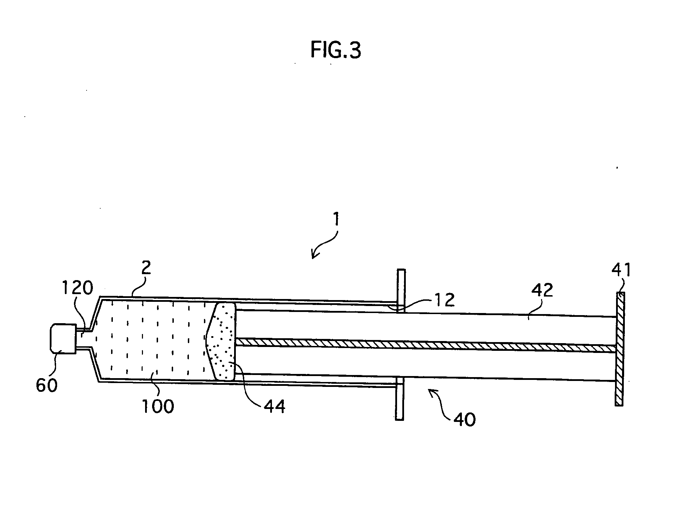

FIG. 3 is a cross-sectional drawing showing the construction of a syringe-type cell handling device 1 of the Third Embodiment of the cell handling device of the present invention. The differences distinguishing the Third Embodiment from the First and Second Embodiments described above are that the syringe main body 2 is constructed in a cylindrical shape resembling the body of a conventional syringe, and that a plunger head 44 is constructed from a gas permeable material. Any of the materials described in the First and Second Embodiments can be used as the gas permeable material.

The plunger head 44 is fixed to the syringe-side tip of the body 42 of the plunger 40 and fits tightly against the internal walls of the syringe main body 2 so as to form a liquid-tight seal therewith. Here, as the plunger head 44 is exclusively permeable by gases, cell suspension solution 100 does not leak to the exterior.

Further, in the Third Embodiment too, constructing both the syringe main body 2 and the plastic head 44 from the above-described cell non-adhesive material is preferable.

With the syringe-type cell handling device 1 of the Third Embodiment of this kind of construction, effects similar to those of the Second Embodiment are achieved.

Further, in the syringe-type cell handling device 1 of the Third Embodiment, since the gas permeable plunger head 44 enables cells in the cell suspension 100 to exchange gases with the exterior while preventing the occurrence of contamination due to bacteria and the like, the cells can be stored satisfactorily with any reduction in cell activation being suppressed. Further, the cell handling device 1 also has the effect of preventing destruction of the cells by bubbles, bubbles contained in the cell suspension 100 being effectively removed through the plunger head 44 to the exterior.

Note that during the operation used to eliminate bubbles from the syringe-type cell handling device 1 of the Third Embodiment, having the leur pointing downwards, the opposite direction to the Second Embodiment, is preferable since, if this is the case, the bubbles in the suspension 100 collect in proximity to the gas permeable film of the plunger head 44, and are easily discharged.

Further, in the Third Embodiment, though an example construction in which the entire plunger head 44 includes a gas permeable material has been indicated, the present invention is not limited to such an arrangement. For example, the plunger head 44 may be constructed by manufacturing a plunger head body from a tensile material similar to those used conventionally, providing through holes in the main surface of the head body, and providing a gas permeable region (a gas permeable film, for instance) so as to cover the formed through holes.

### (Fourth Embodiment)

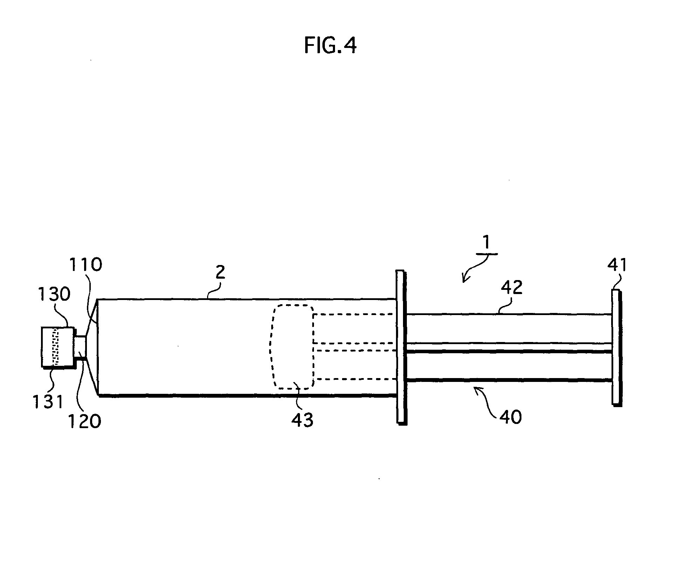

FIG. 4 is an exterior view of the construction of a syringe-type cell handling device 1 of the Fourth Embodiment of the cell handling device of the present invention. The distinguishing characteristics of the Fourth Embodiment are that a syringe body 2 is constructed from the cell non-adhesive material, and that a gas permeable film 131 is provided as a filter in a filter cap 130 fitted to the tip of a leur 120 of the main body 2, with no gas permeable region being provided in the main body 2. Here, the materials described in the First to Third Embodiments can be used as for the gas permeable film (gas permeable material) and the cell non-adhesive material.

In the syringe-type cell handling device 1 of the Fourth Embodiment that has this type of construction, gas is exchanged between the interior and exterior of the cell handling device via the gas permeable film 131 of the cap 130, and consequently, beneficial effects similar to those of the First to Third Embodiments are achieved.

Further, in the syringe-type cell handling device 1 of the Fourth Embodiment, the bubbles in the cell suspension 100 can be satisfactorily removed, as in the Third Embodiment, by pointing the cap 130 upwards and pressing on the plunger 40 to concentrate them in proximity to the leur 121 (and to the gas permeable film 131).

The syringe-type cell handling devices 1 of the Second, Third and Fourth Embodiments are of a construction in which a gas permeable region is provided at the front or back of the direction in which the plunger 40 slides. As a result of the provision of these regions at the front or back directions of the plunger, bubbles can be easily expelled from the gas permeable region as the plunger 40 sliding operation takes place.

Here, the cells can be made to float more satisfactorily in the cell suspension 100 when stored and a smoother cell transplant achieved by making the gas permeable regions 20 and 131, the front section 100, or the plunger head 44, all of which have been described in the First to Fourth Embodiments, both gas permeable, and cell non-adhesive. This is achieved in these components by using either a porous film composed of one of the above-described hydrophobic materials, or by using a gas permeable resin material such as silicone resin, polyethylene, or polystyrene.

### (Example modifications and comparative performance tests for the First to Fourth Embodiments)

In the First to Fourth Embodiments, examples in which the whole of the syringe body 2 is constructed from a cell non-adhesive material have been indicated. However, in this invention, rather than having to construct the whole of the syringe main body 2 from the cell non-adhesive material, it is acceptable to construct only the internal walls in contact with the cell suspension from the cell non-adhesive material. Even if the syringe main body 2 is constructed such that only parts of the inner walls in contact with the cell suspension 100 are constructed from the cell non-adhesive material, a corresponding beneficial effect can be expected. However, in order to fully obtain the beneficial effects of the present invention, it is preferable either to construct, as far as possible, the internal walls of the syringe main body in contact with the cell suspension 100 from the cell non-adhesive material or to construct the whole of the syringe body 2 from the same material.

### Performance Comparison Tests

Here, in order to compare the performance of syringes of conventional technology and syringes of the present invention, cells were cultured in various syringes based on the models (a) to (g) described below, and cell survival rates were measured. Syringes of the Second and Fourth Embodiments were used as examples of the present invention.

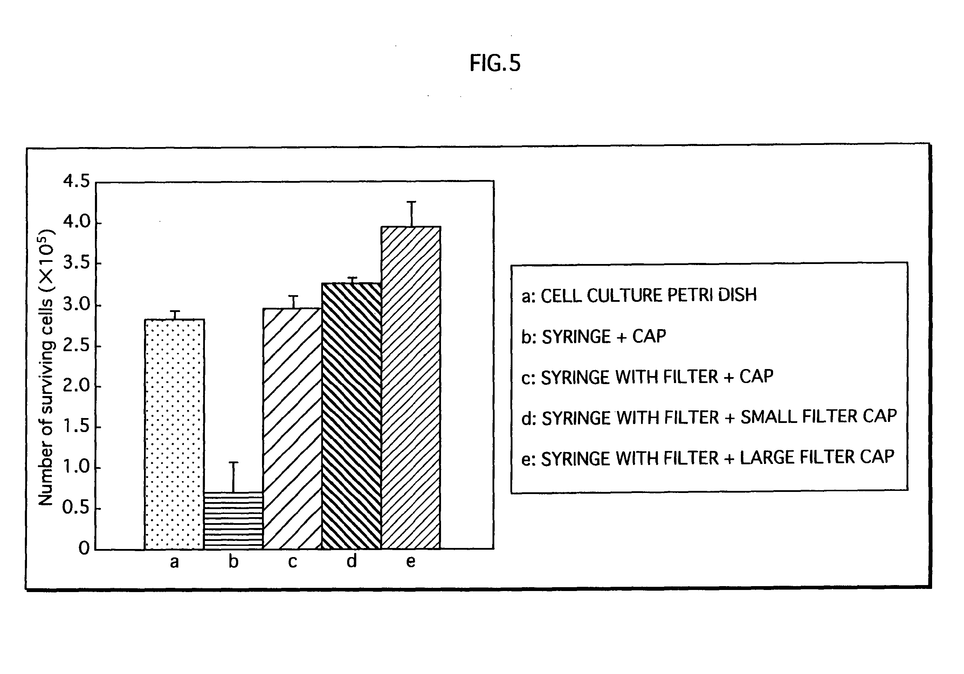

FIG. 5 shows data indicating the results of the tests.
(a) Culture using a suspension cell culture petri dish
(b) Culture using a medical-use syringe of a conventional construction (made from polypropylene) in a sealed state achieved by capping the tip of the leur.
(c) Culture us ing a medical-use syringe of the Second Embodiment (a gas permeable film being provided in the front section, and the syringe body being formed from a cell non-adhesive material) in a sealed state achieved by capping the tip of the leur.
(d) Culture using a medical-use syringe of the Second Embodiment (a gas permeable film being provided in the front section, the syringe body being formed from a cell non-adhesive material), in a state in which the filter cap (small scale) of the Fourth Embodiment is fitted to the end of the leur.
(e) Culture using a medical-use syringe of the Second Embodiment (a gas permeable film being provided in the front section, and the syringe body being formed from a cell non-adhesive material) in a state in which the filter cap (large scale) of the Fourth Embodiment is attached to the end of the leur.

A suspended culture was set up in the suspension cell culture use petri dish (area 21 cm²) of (a).

A filter of e-PTFE manufactured with a thickness of 70 µm and a hole diameter of 0.1 µm was used.

Some other dimensions were as follows.

Gas permeable area of the small scale filter cap: approximately 43 mm²

Gas permeable area of the large scale filter cap: approximately 113 mm²

Syringe volume: 10 mL

Gas permeable area of the front section: approximately 70 mm²

Here, the following method was used as a practical evaluation of the cell survival rate.

In this method concentrated and separated surface marker Leneage negative Scal+sKit+ (hereinafter KSL; suspended cells) of hematopoietic stems cells (HSC) from mouse bone marrow were purified, and in each syringe, 10000 cells were suspended in 2 mL of culture liquid and cultured for three days. Next, The surface markers and colony assay were used to evaluate the number of surviving HSC.
- The following types of culture liquid were used.
   Stem Span medium (Stemcell Technology)
   50 ng/mL murine stem cell factor (mSCF; Peprotech)
   20 ng/mL human filt-3-liganc (Peprotech)
   20 ng/mL murine thrombopoietin (mTPO; Stemcell Technology)
- Type of antibiotic; 0.05 µg/ml Streptomycin

From the test results shown in FIG. 5, it is seen that, whereas there was substantial deactivation in (b) for the conventional medical syringe, the highest performance was obtained in the examples using syringe-types (d) and (e). This superior cell storage performance is thought to be a result of a high gas exchange performance being obtained for syringe of the example by providing a gas permeable film in the front section and by fitting a filter cap onto the leur, and gas being exchanged satisfactorily between the exterior and the cell suspension stored inside the syringe.

Thus, in the present invention, it is anticipated that if the characteristic parts of the cell handling devices of the various embodiments were combined, a very high cell storage performance could be acquired. For example, though the data shows results for a combination of the Second and Fourth Embodiments, it would be possible to give the syringe a larger gas permeable area by providing gas permeable regions in the side of the syringe main body and by making the plunger head gas permeable as in the First and Third Embodiments respectively. This, it is considered, would enable the cells in the cell suspension to exchange gases more satisfactorily with the exterior, and inhibit a fall in cell viability. Further, the ef f iciency of removing bubbles from the cell suspension could be increased, and the cells could be stored more safely without incurring destruction.

Note that, as shown in (c), it is seen that even when a filter cap is not fitted, by providing a gas permeable region in the front section of the syringe main body, cell activation at least substantially the same as, or better than, culture conditions in the cell culture use petri dish can be maintained. On the other hand, if the fall in cell activation in (b) is considered, it is seen that cell storage equivalent to the satisfactory cell storage of the present invention cannot be obtained simply by using a conventional medical syringe as the cell handling device.

From the above observations, it is clear the syringe-type cell handling device indicated in the First to Fourth Embodiments combines the characteristic of enabling the cells to be transplanted easily and quickly at cell transplantation and the characteristic of storing cells very effectively.

### (Fifth Embodiment)

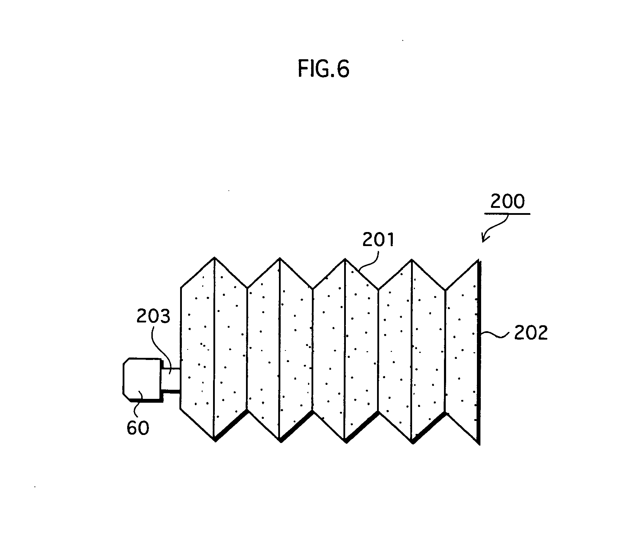

FIG. 6 is an external view showing the construction of a cell handling device 200 of the Fifth Embodiment.

The cell handling device 200 is composed of flexible gas permeable material, and is cylindrical. Its main body surrounding walls 201 have a concertina form, enabling it to expand and contract while maintaining a cylindrical form. A leur 203 is formed on a front end portion of the main body surrounding walls 201, and the internal space within the main body surrounding walls 201 is kept liquid-tight when the device is not being used by tightly fitting a cap 60 onto the leur 203. A cell suspension 100 (not shown in the drawings) is held inside the cell handling device 200.

Any of the gas permeable materials described in the First to Fourth Embodiments can be used to construct the cell handling device 200. However, in the Fifth Embodiment, since the cell handling device is largely constructed from the gas permeable material, the cylindrical form of the cell handling device is maintained by the gas permeable material, and it is therefore considered preferable that a gas permeable material of sufficient strength is used. Note also that when a porous film is used as the material for the cell handling device 200, there are cases in which the transparency of the cell handling device falls, making it harder to check the cell suspension 100 inside. In such cases, the transparency of the cell handling device 200 should be ensured by constructing at least a part thereof (the back end 202 of the main body surrounding walls 210) from a gas permeable resin, enabling the cell suspension 100 held inside the device to be checked. Such a method can may also be used for a cell handling device 300 of the Sixth Embodiment, which is described below.

With the cell handling device 200 of the Fifth Embodiment having the above construction, since the whole of the cell handling device 200 is made of the gas permeable material, the oxygen necessary for cell survival can be taken in across a large area encompassing the entire set of inner walls of the cell handling device 200, while contamination, due to bacteria and the like, of the cell suspension 100 held-tight inside the device,is prevented.Consequently,compared to a cell handling device with only one part formed from the gas permeable material, a far superior gas exchange capability can be obtained. Being of a concertina shape, the cell handling device 200 is easily kept in vessel form during the storage period, and it is possible to ensure that a large cell handling device surface area is contributing to gas exchange for the cells.

Moreover, compared to the constructions of the First to Fourth Embodiments the cell handling device 200 has a structure that is simpler, and has the advantage of being easier to manufacture. Further, as no plunger is required in the cell handling device 200, there is no need to worry about leakage (seal leakage) from the clearance between the body and the plunger during storage.

The superior performance of this kind of cell handling device 200 is also displayed when the device is used in regenerative treatments. By removing the cap 60, attaching a needle or catheter to the leur 203, bringing the device to a predetermined position inside a living body, and simply applying a pressure via the hands or a medical device to the back end 202 of the main body surrounding walls 210, cells can be injected quickly and easily into the living body while the occurrence of contamination avoided. Further, when a porous film is used in a part of the device, an operation to remove bubbles from the cell suspension 100 can be efficiently carried out by pressing on the back end 202.

### (Sixth Embodiment)

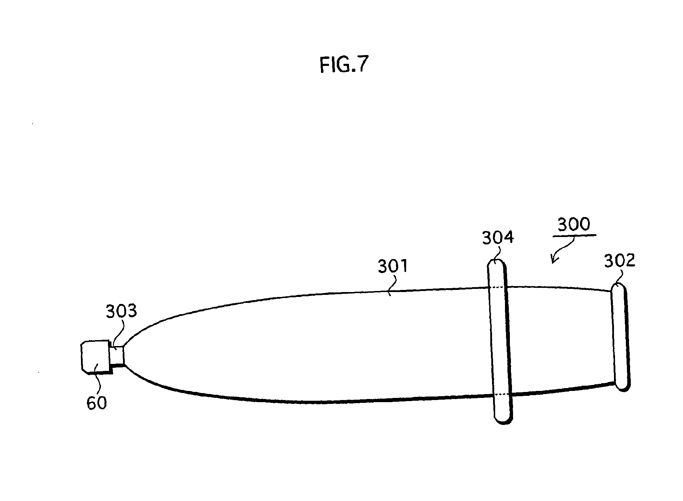

FIG. 7 shows an external view of the construction of a cell handling device 300 of the Sixth Embodiment.

The cell handling device 300 is composed of a gas permeable, flexible material the same as the one used for the cell handling device 200, and has main body surrounding walls 301 which form a long thin bag (tube). A leur 303 is formed in a front end portion of the main body surrounding walls 301, and the space inside the main body surrounding walls 301 is kept liquid-tight when the device is not in use by tightly fitting a cap 60 onto the leur 303. Further, a flat removable ring 304 is fitted to the main body surrounding walls 301 from a back end 302 of the device. A cell suspension 100 (not shown in the drawings) is held, liquid-tight, inside the cell handling device 300.

Using the cell handling device 300 of the Sixth Embodiment, effects similar to the Fifth Embodiment are achieved. However, as a consequence of the difference in structure, and in particular, because there is no need to produce a concertina shape as in the cell handling device 200 of the Fifth Embodiment, the cell handling device 300 has the additional merit of being even simpler to manufacture.

When the cell handling device 300 is used in a regenerative treatment, the cap 60 is first removed and a needle or catheter attached to the leur 303, and the back end 302 of the main body surrounding walls 301 is held. The operator then moves the removable ring 304 towards the front end using his fingers. By simply carrying out this straightforward operation, the removable ring 304 is made to exert a pressure on the main body surrounding walls 301, enabling the cell suspension 100 to be discharged from the leur 303. The beneficial effect of this arrangement, as for the Fifth Embodiment, is to enable cells to be injected quickly and easily into a living body while the occurrence of contamination is avoided.

Further, though in the Fifth and Sixth Embodiments the cell handling device has been described as having a concertina form or long, thin, flexible tube form, the cell handling device of the present invention is not limited to these forms, and may, for example, have a cylindrical form, a bulb form, or a rectangular parallelepiped form, provided it is manufactured from one of the materials described as a construction material for the cell handling devices 200 and 300. These forms are satisfactorybecause, if the cell handling device is provided with a leur, when the device is used, cells can be satisfactorily transplanted into a living body, and speedy regenerative treatment realized.

If, as the material for the cell handling devices 200 and 300 of the Fifth and Sixth Embodiments, either a porous film composed of a hydrophobic material, or a gas permeable material such as silicone resin, polyethylene, polystyrene or the like is used to make the cell handling device 300 cell non-adhesive, cells or clumps of cells can be held more satisfactorily suspended in the cell suspension 100, and transplanted smoothly. Note that these are the same types of materials used in the gas permeable layers 20 and 131, the front section 1100, the plunger head 44 and the cell handling device 200 of the First to Fifth Embodiments.

Note also that in the Fifth and Sixth Embodiments, when gas permeability is not required to any great extent because the cell suspension 100 holding period is extremely short, the cell non-adhesive material of the syringe main body 2 of the First Embodiment can be used for the cell handling device 200 and 300 with a main object of storing the cells or clumps of cells suspended in the culture liquid.

Further, note that the concertina and tube form cell handling devices 200 and 300 of the Fifth and Sixth Embodiments can also be used as bags 50 to be stored in the syringe main body 2 of the Seventh and Eighth Embodiments described below.

### (Seventh Embodiment)

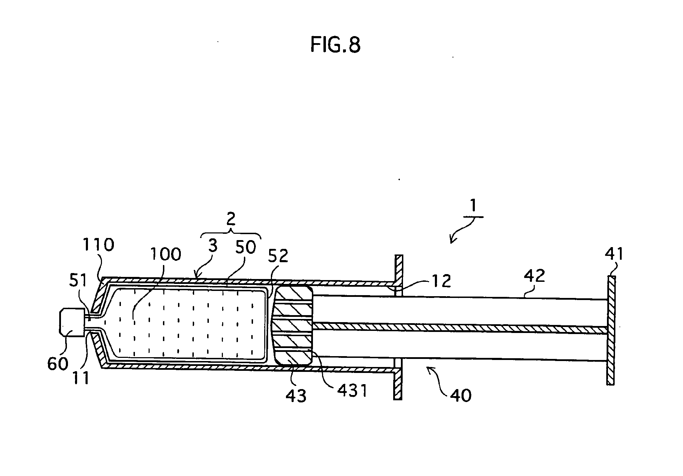

FIG. 8 is a cross-section showing the construction of the syringe-type cell handling device 1 of the Seventh Embodiment.

The syringe-type cell handling device 1 is constructed from a syringe main body 2 composed of a cylindrical body 3 and a bag 50, and a plunger 40.

The cylindrical body 3 and the plunger 40 can be manufactured from materials commonly used for syringes. The cylindrical body 3 is formed to be approximately cylindrical and to have a front section 110 that is disc shaped with a central bore hole 11.

The bag 50 is formed to have a leur 51 at one end and a back end 52 at the other, and is a flexible, cylindrical-bodied reservoir whose volume can be reduced. The bag 50 has a back end 52 disposed opposite a plunger head 43 and forms a pressure part whose volume can be reduced when pressed upon, and the contents it holds are forced out from the leur 51 by pressing in the plunger head 43. Further, the bag 50 is received into the cylindrical body 3 so as to sit against the internal surfaces therein, and in such a way that the leur 51 protrudes through the bore hole 11 to the exterior. With this construction, none of contents remain in the bag 50 when the plunger head 43 is pushed to the front of its range. Note that forming the leur 51 from a material more rigid than the one used for the section storing the cells so that a cap can be tightly fitted and a conduit such as a needle or catheter attached is preferable.

The syringe-type cell handling device 1 of the Seventh Embodiment is of construction in which the cell suspension 100 is held liquid-tight in the bag 50. The principal distinguishing characteristic of the Seventh Embodiment relates to the bag 50. Namely, the bag 50 is constructed from a gas permeable material and is liquid-tight, and consequently, the cells in the cell suspension 100 stored inside the bag 50 do not pass to the exterior except through the leur 51, and gas exchange can take place with the exterior of the bag 50.

As this gas permeable material, any of the gas permeable materials described in the First to Sixth Embodiments can be used.

Further, though the gas permeability required by the bag 50 varies according to factors such as the surface area of the cell handling device, the quantity of cells filling the bag 50, the type of cells contained, and the storage conditions, it is necessary that the bag is sufficiently permeable for cells filling the cell handling device to survive. Thus, portions of the inner parts of the bag 50 should be made gas permeable. To obtain sufficient gas permeability, however, it is preferable to make all of the inner parts of the bag 50, or the entire bag, from the gas permeable material.

When the syringe-type cell handling device 1 of the Seventh Embodiment is filled with the cell suspension fluid 100, an empty bag 50 may be pre-fitted into the cylindrical body 3, and the cell suspension 100 introduced into the bag 50 from the leur 51 tip. Alternatively, the bag 50 may be fitted into the cylindrical body after first being filled with the cell suspension 100. In order make use of a cell handling device which permits removal of the bag 50 from the cylindrical body 3 in this way, it is necessary that (a) the bore hole 11 in the front section 110 is set large enough to enable the leur 51 with attached cap 50 to pass through, (b) the cap 60 is attached after the bag is fitted into the cylindrical body 3, or (c) when inserted into the bore hole 11 of the front section 110, the leur 51 with attached cap is capable of deforming to a size and shape that enable it to pass therethrough.

When the device is not in use, (during cell storage) the leur 51 tip is fitted with the cap 60, and this keeps the inside of the bag, which is liquid-tight.

Further, with an object of ensuring the cap 60 is satisfactorily attached to the leur 51, it is preferable to construct at least the leur 51 portion of the bag 50 from a material that has some degree of strength.

Though the plunger 40 largely resembles the plunger of the First Embodiment, it is characterized by a plurality of holes 431 formed in the plunger head 43 parallel to the axial direction of the syringe. With these holes 431 as circulation paths, the cell suspension 100 inside the bag 50 can exchange gases with the bag 50 exterior (exterior to the plunger head 43).

The syringe-type cell handling device 1 is normally sterilized before use.

In the syringe-type cell handling device 1 of the Seventh Embodiment, which is of this type of construction, the foremost distinguishing characteristic is that the bag is gas permeable while preventing contamination due to the intrusion of bacteria or the like, and the cells included in the cell suspension 100 in the bag 50 can therefore exchange gases with the exterior via the bag 50, and through the holes 431 in the plunger head 43 and the clearance between the bore hole 11 and the leur 51. Consequently, the cells in the cell suspension 100 can take in the oxygen they require to survive from the bag 50 exterior, and the cells can be stored satisfactorily in the bag 50.

Note that though, in the Seventh Embodiment, gas exchange is made possible by using the holes 431 in the plunger head 43 and the bore hole 11 in the front section 110 as circulation pathways, provided gas can be supplied to the cells from the device exterior, the device is by no means limited to using the holes 431 and the bore hole 11, and gas permeable regions may be provided in any other section.

As for materials, the bag 50 of the Seventh and hereafter described Eighth embodiments can be made cell non-adhesive and made to satisfactorily float and store the cells in the cell suspension 100, by using either a porous film composed of a hydrophobic material, or a gas permeable material such as silicone resin, polyethylene, polystyrene or the like, in the same way as for the gas permeable layers 20 and 131, the front section 1100, the plunger head 44 and the cell handling devices 200 and 300 of the First to Sixth Embodiments. If such a material is used, the time and effort as sociated with detaching cells from the cell handling device using pharmaceuticals (detachment agents such as EDTA or trypsin), or by carrying out temperature varying treatments, are no longer required, physical/chemical damage to the cells is prevented, and the efficiency of the cell handling process can be greatly increased. Further, since, in this kind of cell detachment processing, intricate operations such as cell cleaning are not required, a speedy regenerative treatment can be prescribed, and the load on the patient receiving the transplant is lightened.

Further, since the syringe-type handling device 1 of the Seventh Embodiment has, as a second distinguishing characteristic, the makeup of a medical-use syringe, when it is used in a regenerative treatment, operations to transplant cells can be carried out quickly and simply, in the same way as for the syringe-type cell handling device 1 of any of the First to Fourth Embodiments.

Note also that in the syringe-type cell handling device 1 of the Seventh Embodiment, when the bag 50 is constructed from a porous film, setting the pores in the film to a predetermined diameter makes liquid leakage less likely, even when a pressure is applied to the bag. Hence, when the bubbles are removed, the loss through leakage of valuable cell suspension 100 can be prevented. This effect is of particular benefit when the number of cells procured is limited.

Further, when the bag 50 is made from a porous material, it may be the case that the bag whitens, making it difficult to check inside. Transparency in the bag 50 is particularly necessary when checking how much of the cell suspension 100 remains and when checking for the presence of contamination, so when a porous material is chosen, the porosity, the pore diameter, and the like should adjusted appropriately.

In the syringe-type cell handling device 1 of the Seventh Embodiment, at least the cylindrical body 3 and the plunger 40 can be reused as neither of them come into contact with the cell suspension 100.

Further, though in the above example the bag 50 is constructed from a material that is gas permeable, or gas permeable and cell non-adhesive, if the gas permeability requirement is not that important, such as in cases where the storage period is very short, the bag 50 can be constructed from one of the above-described materials that have cell non-adhesiveness as their main object, such as the material used in the construction of the syringe body 3.

### (Eighth Embodiment)

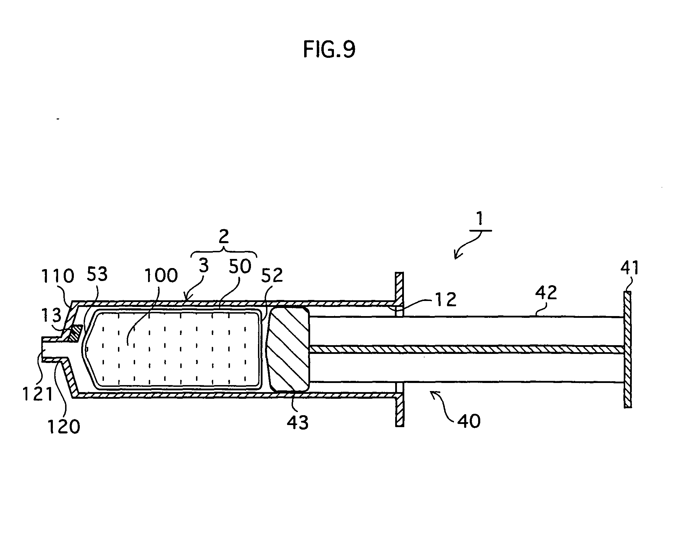

FIG. 9 is a cross-sectional drawing showing the construction syringe-type cell handling device 1 of the Eighth Embodiment.

The differences between the Eighth Embodiment and the Seventh Embodiment are that the holes 431 are not provided in the plunger head 43, that the bag front end 53 is sealed within the cylindrical body 3, and that a bag cutter 13 is provided within the cylindrical body 3 so as to oppose the bag front end 53.

The bag cutter 13 can, in practice, be constructed from a sharp metal blade or needle. In FIG. 9, an example in which a metal blade is provided as the bag cutter 13 is indicated. However, when it is necessary to consider the disposal of the device, the bag cutter 13 can also be constructed, for example, from a resin member of the same material as the syringe main body.

During cell storage, substantially the same beneficial effects are achieved using the syringe-type cell handling device 1 of the Eighth Embodiment as are achieved using the syringe-type cell handling device 1 of the Seventh Embodiment.

Moreover, in the syringe-type cell handling device 1 of the Eighth Embodiment, because, as shown in FIG. 9, the front end 53 of the bag 50 is exposed to the atmosphere via the opening 121 in the leur 120 provided in the front section 110 as a circulation pathway, in comparison to the Seventh Embodiment, gas exchange and bubble removal can be carried out more favorably.

### (Cell Handling Device Modifications)

The forms of cell handling device described above for the First to Eighth Embodiments are, of course, no more than illustrative examples, and the cell handling device may take other forms. Any form is acceptable as long as the cell handling device is capable of storing cells in an internal section that is liquid-tight, and provided that at least a portion of the inner walls of the cell handling device in contact with the cells is constructed from a cell non-adhesive material or a gas permeable material.

For example, the cell handling device of the present invention may be constructed to look cylindrical when viewed externally. In such a case, it would be possible to store cells inside the cell handling device and attach a needle or catheter to one side thereof, and to discharge the cells by reducing its volume.

### 2. Tissue regeneration composition of the present invention

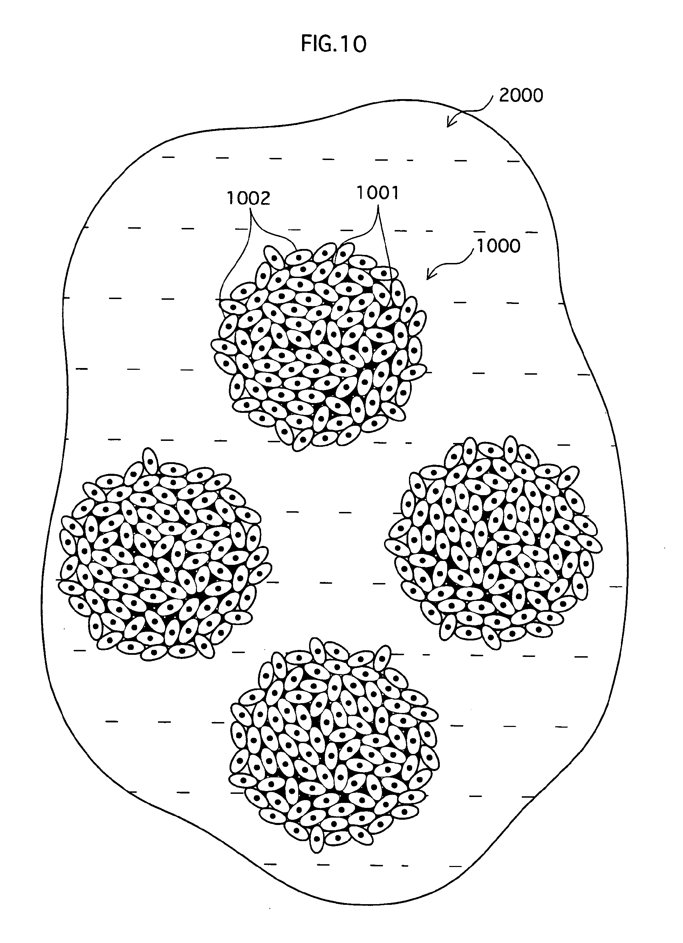

FIG. 10 is a partial enlargement showing the tissue regeneration composition of the present invention. This tissue regeneration composition includes cell culture microcarriers 1000 and a fluidity medium 2000 (a culture liquid, for instance) containing these culture microcarriers.

The tissue regeneration composition can, as described below, be used instead of a conventional regeneration composition that contains larger scale scaffolds with cells disposed thereon, and has properties ideally suited to a tissue regeneration composition for regenerative treatments.

As shown in FIG. 10, the cell culture microcarriers 1000 are dispersed in the fluidity medium 2000, and constitute a plurality of cells 1002 adhering to the surface of scaffold microcarriers 1001, which are formed from a material that is bioabsorbable. Further, the number of adhering cells 1002 varies between the individual scaffolds.

For the scaffold microcarriers 1001, a diameter of between 10 µm and 2000 µm inclusive is favorable, and between 50 µm and 500 µm inclusive is particularly favorable. Setting the microcarrier diameter within this range causes the cell culture microcarriers 1000 to be favorably dispersed in the fluidity medium 2000, and on account of this, the tissue regeneration composition as a whole has fluidity. Consequently, if a tissue regeneration composition containing the cell culture microcarriers 1000 is stored in any of the cell handling devices of the above described First to Eighth Embodiments, it can be favorably discharged from inside the cell handling device to the device exterior via a leur or the like. Further, in order ensure that the adherence area for the cells is sufficient, it is preferable that the cell culture microcarriers 1000 are porous with pores of a sufficient diameter to allow the invasion of cells.

A number of well-known materials can be used as the bioabsorbable material that constitutes the scaffoldmicrocarriers 1001. For example, it is possible to select one or more of a group of materials that includes aliphatic polyester, polylactic acid, polyglycolic acid, lactic acid-glycolic acid copolymer, lactic acid caprolactam copolymer, glycolic acid-carbonate copolymer, polydioxanone, chitosan, cross-linked hyaluronic acid, alginic acid, collagen, laminin, fibronectin, vitronectin, polylysene, fibrin, calcium phosphate, calcium carbonate, polycyanoacrylate, polyglutamic acid, polyhydroxybutyrate,polymalicacid,polyanhydride,polyorthoester, chitin, starch, fibrinogen, hydroxyapatite and gelatine. These materials may be used independently, or in combination with other materials from the list.

One example of a possible manufacturing method for the scaffold microcarriers 1001 involves forming a particulate by spraying a solution containing the bioabsorbable material dissolved in a solvent, and removing the solvent by evaporation. In another possible manufacturing method a solution of the material is dispersed in a dispersion medium to form an emulsion and the dispersion medium is subsequently evaporated. Alternatively, the raw materials for the bioabsorbablematerial can be emulsified and the emulsion subsequently polymerized using emulsion polymerization.

A example method for making the scaffold microcarriers porous is a freeze drying in which the bioabsorbable solution is emulsified and frozen, and the solvent subsequently evaporated. There is also method in which microcarriers are formed from a mixture containing the bioabsorbable material and powdered salt or powdered sugar as a pore forming agent, which is subsequently dissolved and removed to produce porous microcarriers.

As for the cells 1002, any of the various types of cells described above can be selected depending on the aim of the treatment. There is no particular limit to the types of cells, but some examples of possible cell types are included below. Apart form stem cells such as embryonic stem cells (ES cells), embryonic germ cells (EG cells), adult stem cells (AS cells), mesenchymal stem cells, neural stem cells, endothelial stem cells, hematopoietic stem cells, and hepatic stem cells, differentiated cells such as bone cells, chondrocytes, muscle cells, heart muscle cells, nerve cells, tendon cells, fat cells, pancreatic cells, heptocytes, liver cells, hairfollicle cells, blood cells and the like can also be used. Thus, various types of cells including embryonic stem cells, stem cells at various differing stages of differentiation, and cells that have differentiated into various different tissues can be used. When adhesive cells are selected from among the above, the composition is effective because it is able to offer scaffolds for proliferation and differentiation. Here, the term "adhesive cells" includes the vast majority of cells with the exception of hemocyte-type cells of the hematopoietic set of stem cells.

Further, genetically modified cells formed by modifying any of the given cell types via a genetic engineering method can also be used without difficulty.

Moreover, as the fluidity medium 2000, a medium other than regular culture fluid, such as physiological saline solution, a phosphate buffer solution or the like can be used. If this case, however, the culture liquid used in the cell culture must be replaced with the other medium.

In order to make the cell culture microcarriers 1000, the cells 1002 in the culture liquid (fluidity medium 2000) should first be seeded on the scaffoldmicrocarriers 1001, and cultured in the culture liquid. This causes the cells 1002 to adhere naturally to the surface of the scaffold microcarriers, and the cell culture microcarriers 1000 are formed. The cell culture can be carried out using known methods.

An example of a cell culture procedure is as follows. Firstly the cells are isolated from a living body and purified, and the target cells are selected. Next the cell culture microcarriers are added, growth factor is added as necessary to start proliferation or to induce differentiation into a standard cell type, and the cells are cultured while being slowly stirred. In this case, the growth factor or the like, which is the constituent necessary for the cells to proliferate, may be contained in the cell culture microcarriers.

Here, it is desirable to implement the actual cell culture in an incubator. The obtained tissue regeneration composition is introduced into a predetermined cell handling device (one of the cell handling devices of the First to Eighth Embodiments of the present invention is preferable), and stored therein under appropriate storage conditions until needed for a treatment. Though storing the cells at low temperature is preferable, if conditions are such that cell deactivation is unlikely, they can also be stored at normal or higher temperatures.

According to this type of method, the cells 1002 adhere to the inside of the pores in the porous surface of the scaffold microcarriers 1001, forming cell culture microcarriers 1000. The cells 1002 are cultured in a satisfactory manner in these cell culture sites or differentiation inducement sites.

Further, in order to improve the adhesiveness of the cells 1002 to the scaffold microcarriers 1001, it is preferable to treat the surface of the scaffoldmicrocarriers to improve cell adhesiveness via a known method (a physical treatment such as Ozone treatment, UV treatment or plasma treatment, a chemical treatment such as hydrochloric acid treatment or sulfuric acid treatment, or a coating treatment). Examples of materials used in such a coating include laminin, fibronectin, vitronectin, polylysene, fibrin (including preclotting), fibrinogen, gelatine and collagen. Furthermore, the microcarrier itself may be made to absorb a physiologically active material with a fixed composition such as a hormone or one of various types of growth factor. With this construction, the mechanical strength of the scaffold microcarriers 1001 themselves is favorable, and both the adhesiveness of the cells 1002 and the results of the treatment can be improved.

### (Effects of granular tissue regeneration composition)

According to a tissue regeneration composition of the above form, the cells 1002 can be caused to proliferate, or differentiate into target cell types, on the surface of the scaffold microcarriers 1001. During this period, however, the cells 1002 together with the scaffold microcarriers 1001 form minute cell culture microcarriers 1000, which float in the culture liquid, and the composition as a whole has fluidity. When the composition is subsequently used in a regenerative treatment, the cells can be transplanted by injecting the cell culture microcarriers 1000 in their existing state into a living body.

Moreover, as the scaffold microcarriers 1001 are formed from bioabsorbable material, if the cell culture microcarriers are 1000 are stored in one of the syringe-type cell handling devices described in the First to Eighth Embodiments, at use, the cell culture microcarriers 1000 can be smoothly transplanted together with the fluidity medium 2000 into the living body, via a leur or similar part.

Various cell transplantation techniques depending on this kind of syringe-type cell handling devices are conceivable, including the injection cells into a treatment target area without an implanted scaffold, the repeated injection of cells to a scaffold implanted in advance in the treatment target area, and the like. The latter technique is effective where gradual regenerative treatment of a treatment target area is desired (such as in cartilage regeneration, breast regeneration, and the like).

Whereas conventionally cells cannot be straightforwardly removed when they are stored in a vessel because they adhere easily to the inner walls, if the cells are stored in the above described syringe-type cell handling device, they do not adhere to the inner walls while they are stored. Instead, the cell culture microcarriers 1000 are maintained in a floating state in the fluidity medium 2000. Consequently, the cell culture microcarriers 1000 can be injected into a living body by connecting a needle or intravascular catheter to the leur of a cell handling device and pushing in the plunger or the like.

Further, using the syringe-type cell handling device, the cells can be cultured on the scaffold microcarriers 1001, and the cell culture microcarriers 1000 formed due to this cell culture can be injected into a living body from inside the cell handling device via a syringe operation.

Because the scaffold microcarriers 1001 are absorbed into the living body and disappear a predetermined period injection, surgery to remove the scaffolds after the transplant is unnecessary. Because of this, the load on the patient can be markedly reduced, and, in particular, the load on patients, such as infant and elderly patients, who are lacking in physical strength, can be lightened.

Further, if the granular tissue regeneration composition is stored in one of the cell handling devices cited in the First to Eighth Embodiments, the cells can be stored satisfactorily, while cell adhesion to the inner walls of the cell handling device, cell deactivation, and cell destruction due to bubbles are prevented.

The tissue regeneration composition of the present invention can, by injection to the appropriate part of the body, be applied in regenerative treatments for various medical conditions and diseases including osteoarthritis, rheumatoid arthritis, pseudoarthrosis, periodontal disease, progressive muscular dystrophy, heart disease, strokes, Parkinson's disease, spinal cord damage, tendon damage, diabetes, liver damage, digestive organ dysfunction, skin damage, leukemia, vascular disease and the like.

More specifically: in the treatment of an osteoarthritis patient, chondrocytes or their progenitor cells are injected; in the treatment of a patient with periodontal disease, bone cells or their progenitors are injected; in the treatment of a patient with Parkinson's disease, nerve cells or their progenitors are injected; in the treatment of a patient with heart disease, muscle cells or their progenitors are injected; and so on.

Further, though the above explanation describes examples in which the cells used were of a type suitable for regenerative treatment, cells for treatments besides regenerative treatments, or other types of cells may also be used in the cell handling device and composition of the present invention.

### Industrial Applicability

The cell handling device and tissue regeneration composition of the present invention, can be applied, via the injection of stored cells into the affected part or into blood vessels, in treatments for osteoarthritis, rheumatoid arthritis, pseudoarthrosis, progressive muscular dystrophy, myocardial infarction, strokes, Parkinson's disease, spinal cord damage, tendon damage, diabetes, liver damage, digestive organ dysfunction, skin damage, leukemia, vascular disease, hair regeneration, and the like.

## Claims

1. A cell handling device including a vessel able to hold, in a liquid-tight state, a handling medium that is fluid and contains cells, and being able to transfer the handling medium between an interior and an exterior of the vessel via a mouth being opened in the vessel to end the liquid-tight state, the mouth connecting the interior and the exterior, wherein
at least part of the vessel that contacts the handling medium when the vessel holds the handling medium is a gas permeable region for passing a quantity of gas necessary for survival of the cells.

2. The cell handling device of Claim 1, wherein
a whole of the vessel that contacts the handling medium when the vessel holds the handling medium is the gas permeable region.

3. The cell handling device of Claim 1 or 2, wherein
in terms of an overall oxygen permeability quantity, a gas permeability of the gas permeable region is one of 1 mL/24 hr atm or more and 10 mL/24 hr atm or more.

4. The cell handling device of any of Claims 1, 2 and 3, wherein
the gas permeable region is composed of one of a gas permeable resin and a porous film.

5. A tissue regeneration method in which the cell handling device of any of Claims 1 to 4 is used, the tissue regeneration method comprising:
a first step of holding, the handling medium in the vessel; and
a second step of transplanting the handling medium into a living body.

6. The cell handling device of Claim 1, further including a volume varying means for varying a volume of the vessel wherein,
as the volume varying means varies the volume, the handling medium is discharged, or flows into, the vessel.

7. The cell handling device of Claim 1, wherein
the vessel is at least partially composed of a main body that combines with a plunger to form a syringe type device,
the plunger is slidably insertable into the main body, the handling medium being transplanted into a living body by a pushing force being applied to the plunger, and
at least part of the main body and/or the plunger is the gas permeable region.

8. The cell handling device of Claim 7, wherein
the main body is composed of a flexible bag-type vessel that holds the handling medium and deforms as the plunger slides, and a cylindrical exterior part that holds the bag-type vessel, and
at least part of the bag-type vessel is the gas permeable region.

9. The cell handling device of Claim 8, wherein
the bag-type vessel is detachable from the cylindrical exterior part, and handling of the handling medium is possible when the bag-type vessel is in a detached state.

10. The cell handling device of Claim 8 or 9, wherein
the bag-type vessel includes a discharge part for discharging the handling medium and a push part that causes a pushing force to act on the bag-type vessel.

11. The cell handling device of Claim 10, wherein
except for the discharge part, a whole of the bag-type vessel is composed of a flexible material that is contractible under the pushing force.

12. The cell handling device of Claim 11, wherein
the bag-type vessel includes a concertina section, and
the concertina section is shortened via the push part effecting the pushing force.

13. The cell handling device of Claim 11, wherein
the bag-type vessel is a tube.

14. The cell handling device of Claim 8, wherein
a gas permeable region is provided in at least one other part besides the bag-type vessel so as gas exchange between a handling device exterior and the bag-type vessel is possible when the bag type vessel is being stored in the cylindrical exterior part.

15. The cell handling device of Claim 8, further including a rupturing means for rupturing the bag-type vessel when the bag-type vessel is being stored in the cylindrical exterior part.

16. The cell handling device of Claim 8, wherein
a discharge part that discharges the handlingmedium in a plunger forward-sliding direction is provided in the main body, and
the discharge part is formed such that a needle, an intravascular catheter or other conduit can be connected thereto.

17. The cell handling device of any of Claims 6, 7, 8, 9, 10, 11, 12, 13, 14, 15, and 16, wherein,
in terms of an overall oxygen permeability quantity, a gas permeability of the gas permeable region is one of 1 mL/24 hr atm or more and 10 mL/24 hr atm or more.

18. The cell handling device of any of Claims 6, 7, 8, 9, 10,
11, 12, 13, 14, 15, 16 and 17, wherein,
the gas permeable region is composed of one of a gas permeable resin and a porous film.

19. A tissue regeneration method in which the cell handling device of any of Claims 6, 7, 8, 9, 10, 11, 12, 13, 14, 15, 16, 17, and 18 is used, the tissue regeneration method comprising:
a first step of holding the handling medium, in the vessel; and
a second step of transplanting the handling medium into a living body.

20. The cell handling device of Claim 7, wherein
portions of the gas permeable region are provided at a plurality of separate locations in the main body and each portion extends in a sliding direction of the plunger.

21. The cell handling device of Claim 20, wherein
each portion of the gas permeable region is composed of a material whose gas permeability is higher than a gas permeability of a principal material of the main body.

22. The cell handling device of Claim 7, wherein
a portion of gas permeable region is located in the main body in a sliding direction of the plunger.

23. The cell handling device of Claim 22, wherein
a portion of the gas permeable region is formed at a tip of the plunger.

24. The cell handling device of Claim 22, wherein
in the main body, a portion of the gas permeable region is located in proximity to a discharge part that is for discharging the handling medium from the main body.

25. The cell handling device of Claim 24, wherein
the discharge part is formed at or in proximity to a surface that makes contact with the plunger when the plunger is in a fully pressed state, and
a portion of the permeable region is formed in the surface.

26. The cell handling device of Claim 24, wherein
a portion of the gas permeable region is provided in a closing member that covers the discharge part.

27. The cell handling device of any of Claims 20, 21, 22, 23, 24, 25, and 26, wherein
in terms of an overall oxygen permeability quantity, a gas permeability of the gas permeable region is one of 1 mL/24 hr atm or more and 10 mL/24 hr atm or more.

28. The cell handling device of any of Claims 20, 21, 22, 23, 24, 25, 26, and 27, wherein
the gas permeable region is composed of one of a gas permeable resin and a porous film.

29. A tissue regeneration composition including cells, a fluidity medium, and granular cell scaffold microcarriers which are scaffolds for the cells, wherein
the cell scaffoldmicrocarriers are composed of a bioabsorbable material and are cell adhesive.

30. The tissue regeneration composition of Claim 29, wherein
a diameter of the scaffold microcarriers is between 10 µm and 2000 µm inclusive.

31. The tissue regeneration composition of Claim 29 or 30, wherein
the scaffold microcarriers are porous.

32. The tissue regeneration composition of any of Claims 29, 30 and 31, wherein
the scaffold microcarriers have undergone a treatment to improve a cell adhesiveness thereof.

33. The tissue regeneration composition of any of Claims 29, 30 31, and 32, wherein
the cells are cells selected from a group of adhesive cells which require scaffolds in order to proliferate.

34. A tissue regeneration composition-containing cell handling device including a vessel able to hold, in a liquid-tight state, a tissue regeneration composition that is fluid and contains cells, and being able to transfer the tissue regeneration composition between an interior and an exterior of the vessel via a mouth being opened in the vessel to end the liquid-tight state, the mouth connecting the interior and the exterior, wherein
the tissue regeneration composition includes i) cells, ii) a fluidity medium, iii) granular cell scaffold microcarriers that are composed of a bioabsorbable material and float in the fluidity medium, and
at least part of the vessel that contacts the tissue regeneration composition when the vessel holds the tissue regeneration composition is a gas permeable region for passing a quantity of gas necessary for survival of the cells.

35. The tissue regeneration composition-containing cell handling device of Claim 34, wherein
a whole of the vessel that contacts the tissue regeneration composition when the vessel holds the tissue regeneration composition is the gas permeable region.

36. The tissue regeneration composition-containing cell handling device of Claim 34, further including a volume varying means for varying a volume of the vessel wherein,
as the volume varying means varies the volume, the tissue regeneration composition is discharged, or flows into, the vessel.

37. The tissue regeneration composition-containing cell handling device of Claim 34, wherein
the vessel is at least partially composed of a main body that combines with a plunger to form a syringe type device,
the plunger is slidably insertable into the main body, the tissue regeneration composition being transplanted into a living body by a pushing force being applied to the plunger, and
at least part of the main body and/or the plunger is the gas permeable region.

38. The tissue regeneration composition-containing cell handling device of Claim 34 or Claim 37, wherein
the cell scaffold microcarriers are more cell adhesive than both the gas permeable region and the vessel that contacts with the tissue regeneration composition.

39. The tissue regeneration composition-containing cell handling device of Claim 38, wherein
a discharge part that discharges the tissue regeneration composition in a plunger forward-sliding direction is provided in the main body, and
the discharge part is formed suchthat a needle, an intravascular catheter or other conduit can be connected thereto.

40. The tissue regeneration composition-containing cell handling device of Claim 38, wherein
in the main body and/or the plunger, at least parts that contact the tissue regeneration composition are formed from a material that is cell non-adhesive.

41. The tissue regeneration composition-containing cell handling device of any of Claims 34, 35, 36, 37, 38, 39, and 40, wherein
in terms of an overall oxygen permeability quantity, a gas permeability of the gas permeable region is one of 1 mL/24 hr atm or more and 10 mL/24 hr atm or more.

42. The tissue regeneration composition-containing cell handling device of any of Claims 34, 35, 36, 37, 38, 39, 40, and 41, wherein
the gas permeable region is composed of one of a gas permeable resin and a porous film.

43. The tissue regeneration composition-containing cell handling device of any of Claims 40, 41, and 42, wherein
the cell non-adhesive material is oneof a hydrophilic material, a hydrophobic material and a material having a negative charge.

44. A tissue regeneration method in which the tissue regeneration composition-containing cell handling device of any of Claims 34, 35, 36, 37, 38, 39, 40, 41, 42, and 43 is used, the tissue regeneration method comprising:
a first step of holding the tissue regeneration composition in the vessel; and
a second step of transplanting the tissue regeneration composition into a living body.
